(19) Europäisches Patentamt

European Patent Office

Office européen des brevets

(11) Publication number: **0 364 490 B1**

## (12) EUROPEAN PATENT SPECIFICATION

(45) Date of publication of patent specification: **10.03.93** (51) Int. Cl.⁵: **C12Q 1/68**, C07H 21/00

(21) Application number: **88906295.6**

(22) Date of filing: **27.05.88**

(86) International application number:
**PCT/US88/01809**

(87) International publication number:
**WO 88/09387 (01.12.88 88/26)**

Consolidated with 88304900.9/0293266
(European application No./publication No.) by
decision dated 08.08.91.

(54) **A method for detecting a retinoblastoma gene defect in tumors using a retinoblastoma gene probe.**

(30) Priority: **29.05.87 GB 8712646**

(43) Date of publication of application:
**25.04.90 Bulletin 90/17**

(45) Publication of the grant of the patent:
**10.03.93 Bulletin 93/10**

(84) Designated Contracting States:
**AT BE CH DE ES FR GB GR IT LI LU NL SE**

(56) References cited:
**EP-A- 0 259 031**

**SCIENCE, vol. 235, no. 4794, 13 March 1987, Lancaster, PA, US; W.-H.LEE et al."Human retinoblastoma susceptibility gene: cloning, identificationd, and sequence" pages 1394-1399**

(73) Proprietor: **RESEARCH DEVELOPMENT FOUN-DATION**
**402 North Division Street**
**Carson City Nevada 89703(US)**

(72) Inventor: **FUNG, Yuen Kai T.**
**2455 Ivanhoe**
**Los Angeles, CA 90039(US)**
Inventor: **T'ANG, Anne**
**2455 Ivanhoe**
**Los Angeles, CA 90039(US)**
Inventor: **MURPHREE, A. Linn**
**2489 Moreno Drive**
**Los Angelos,CA 90039(US)**
Inventor: **BENEDICT, William F.**
**21 East Wedgewood Glen**
**The Woodlands, TX 77381(US)**

NATURE, vol. 305, 27 October 1983, London, GB; W.K.CAVENEE et al."Expression of recessive alleles by chromosomal mechanisms in retinoblastoma" pages 779-784

PROCEEDINGS OF THE NATL. ACADEMY OF SCIENCES USA, vol. 85, August 1988, Washington, DC, US; E.Y.-H.P.LEE et al."Molecular mechanism of retinoblastoma gene inactivation in retinoblastoma cell line Y79" pages 6017-6021

SCIENCE, vol. 236, 26 June 1987, Washington, DC, US; FUNG, Yuen-Kai T. et al."Structural Evidence for the Authenticity of the Human Retionblastoma Gene" pages 1658, 1660

NATURE, vol. 323, 16 October 1986, London, GB; S.H.FRIEND et al."A human DNA segment with properties of the gne that predisposes to retinoblastoma osteosarcoma" pages 644, 646

CHEMICAL ABSTRACTS, vol. 106, no. 7, 16 February 1987, Columbus, Ohio, US; H.SCHEFFER et al."A straight forward approach to isolate DNA sequences with potential linkage to the retionblastoma locus" page 167, column 2, abstract no. 44 946z

CHEMICAL ABSTRACTS, vol. 105, no. 9, 01 September 1986, Columbus, Ohio, US; Y.NAKAGAWA et al."Cloning and characterization of a cDNA specific for bovine retina" page 166, columns 1 + 2, abstract no. 73 577q

CHEMICAL ABSTRACTS, vol. 102, no. 7, 18 February 1985, Columbus, Ohio, US; M.LALANDE et al."Isolation of human chromosome 13-specific DNA sequences cloned from flow sorted chromosomes and potnetially linked to the retinoblastoma locus" page 164, columns 1 + 2, abstract no. 57 143c

CHEMICAL ABSTRACTS, vol. 107, no. 5, 03 August 1987, Columbus, Ohio, US; A.M.V.DUNCAN et al."Reevaluation of the sublocalization of esterase D and its relation to the retionblastoma locus by in situhybridization" page 188, columns 1 + 2, abstract no. 34 221r

⑭ Representative: **Wilkinson, Stephen John et al Stevens, Hewlett & Perkins 1 St. Augustine's Place Bristol BS1 4UD (GB)**

## Description

### FIELD OF THE INVENTION

This invention relates to the field of retinoblastoma gene probes and their use in detecting tumor with a retinblastoma gene defect. Examples of these tumors are fibrosarcoma, soft tissue sarcoma, small-cell carcinoma, melanoma and mammary tumors.

### BACKGROUND OF THE INVENTION

The retinoblastoma (Rb) gene is the prototype for a class of recessive human cancer genes in which loss of activity of both normal alleles is thought to be associated with tumorigenesis. Retinoblastoma (Rb) occurs in both hereditary and nonhereditary forms and is the most common childhood cancer involving the eye. Sporadic bilateral cases are always hereditary, while appropriately 85% of sporadic unilateral cases are nonhereditary. On the basis of statistical analysis of clinical data, it has been proposed that the development of retinoblastoma requires two mutational events. In hereditary cases, where the germline mutation is present in all cells, a second random somatic mutation in a target retinoblast is postulated to be essential for tumorigenesis.

The initial clue to the nature of the first mutational event came from cytogenetic studies of lymphocytes or fibroblasts from patients with retinoblastoma. A small subgroup of these patients was found to have a deletion involving chromosomal region 13q14. Initial tumor cytogenetic data also implicated chromosome 13 as the target for the second mutational event. A loss or partial deletion of chromosome 13, including 13q14 was frequently found in retinoblastomas from patients with two apparently normal constitutional chromosome 13s.

Additional evidence that the second event also involved the Rb susceptibility locus in the 13q14 region came from restriction fragment length polymorphism (RFLP) studies. By a comparison of the normal constitutional and the tumor genotypes, as defined by RFLPs on chromosome 13, it was shown that the reduction to hemizygosity or homosygosity for all or part of an allele of chromosome 13 is a common event in the development of retinoblastoma. Similarly, in osteosarcoma, a bone tumor which occurs frequently as an additional primary malignancy in patients with hereditary retinoblastoma, the development of homozygosity or hemizygosity in chromosome 13 has been observed. All the available data suggest that the loss of activity of both normal alleles at the RB locus is the key event in tumor development. However, it has not been possible until now to prove this hypothesis with certainty and to use this knowledge to detect tumors which have a defective Rb gene and to determine the propensity to develop retinoblastoma.

Nature, 323 (1986) pages 643-646, discloses a 4.7 kb cDNA segment and a 0.9 kb subfragment thereof which hybridize to DNA extracted from retinoblastoma tissue and DNA extracted from osteosarcoma tissue.

Science, 235 (1987) pages 1394-99, discloses the sequence of the retinoblastoma gene and further discloses that transcription of this gene was abnormal in retinoblastomas examined.

EP-A-0259031 describes the use of a cDNA probe for diagnosing retinoblastoma, osteosarcoma and some undifferentiated tumors of unknown cellular origin.

### SUMMARY OF THE INVENTION

An object of the present invention is a method for detecting tumors with a defect in the retinoblastoma gene.

Thus, the present invention provides a method of detecting tumors containing a retinoblastoma gene defect, comprising the steps of:

hybridizing a retinoblastoma gene probe to DNA extracted from a target tissue; and

determining binding of the probe to said DNA, wherein said tumors are selected from the group consisting of fibrosarcoma, soft tissue sarcoma, melanoma, small-cell carcinoma of the lung and breast carcinoma. The target tissue is non-tumor tissue and can be fetal tissue, as well as, post-natal to adult tissue.

The present invention is useful in detection of a wide variety of tumors which have a defect in the retinoblastoma gene.

BRIEF DESCRIPTION OF THE DRAWINGS

The invention will be more readily understood from a reading of the following specification and by reference to the accompanying drawings forming a part thereof:

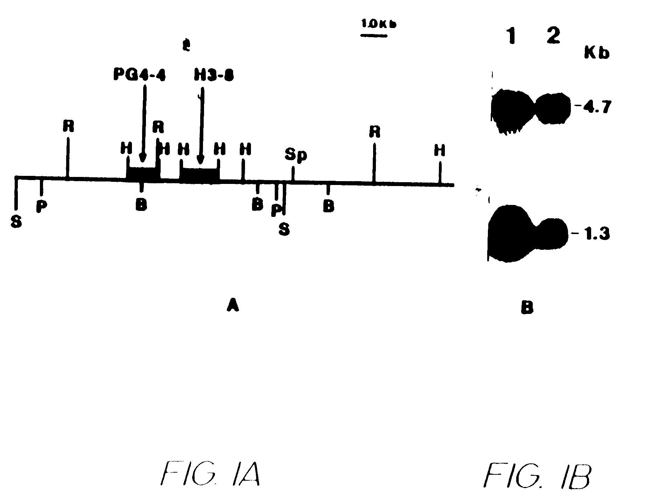

Figure 1A is a restriction map of the Rb locus at the H3-8 region. EcoR1, Hind III, Bgl II, Xba 1 and Sac I restriction fragments from the genomic DNA of a normal human fibroblast (WSI) were cloned into sites in Charon 21A, CH17 or λgtWESλB. Restriction map of the Rb locus at the H3-8 region. An EcoR1 7.7 kb clone was isolated from a genomic library constructed by digesting to completion the genomic DNA of a normal human fibroblast WSI with EcoR1 and cloned into the EcoR1 site of the bacteriophage lambda vector Charon 21A. Restriction fragments free of repetitive sequence obtained close to the ends of this clone were in turn used as probes to screen a genomic library of Hind III fragments from WSI. Similarly, overlapping clones were screened from genomic libraries constructed from completely digested WSI genomic DNA with Bgl II, or Sac 1. Restriction enzyme sites are indicates as follows: R, EcoR1; S, Sac 1; P, Pst1; B, Bgl II; H, Hind III; Sp, Sph 1.

Figure 1B is a Northern blot analysis of immortalized human fetal retinal cells. Polyadenylated RNA were isolated from the human fetal retinal cell lines Ad5/HER B90 and Ad12/HER 8.24 immortalized by transfection with DNA from adenovirus 5 and adenovirus 12 respectively. 10 μg of each of the isolated RNA were separated by 1% agarose gel electrophoresis in 2.2 M formaldehyde and transferred to genescreen membranes. $^{32}_{P}$ labelled complementary RNAs were synthesized according to standard protocols using Bam H 1 linearized pG4-4 and pESD, a subclone of a 1.3 kb human esterase cDNA clone. Random primer probes were synthesized using a standard method. Hybridization was done at 42°C in 50% formemide, 3xSSC, 1x Denhardt, 0.1 M Hepes pH 7.4, 0.05% SDS. Washing was done at 2xSSC at room temperature for 20 minutes followed by 0.1XSSC, 0.1% SDS at 65°C twice for one hour each. The 4.7 kb transcript was detected with the pG4-4 probe and the 1.3 kb with pESD probe.

Figure 2A is a restriction map of the 4.6 kb cDNA of the Rb gene. 4.6 kb cDNA clone was isolated from a cDNA library. Restriction enzyme sites indicated as follows: R1, EcoR 1; Hp, Hpa 1; EV, EcoR V; Pv, Pvu II; Bg, Bgl II; Nd, Nde I; Nc, Nco I; Ps, Pst 1; H III, Hind III.

Figure 2B shows the linear order of the Hind III restriction fragments detected with the various cDNA probes. Each of the boxes represents a discreet Hind III fragment, the size (kb) of which is indicated by the number in the box.

Figure 3A is a Southern blot analysis of Hind III genomic DNA from normal cells, retinoblastoma and their constitutional cells which have been hybridized to pGH2, which is a subclone of the Eco R1 0.9 kb fragment at the 5' end of the cDNA clone.

Figure 3B is a Southern blot analysis of Hind III genomic DNA from normal cells, retinoblastoma and their constitutional cells hybridized to a small pG 3.8m, which is a subclone of the Eco R1 3.8 kb fragment of the cDNA clone.

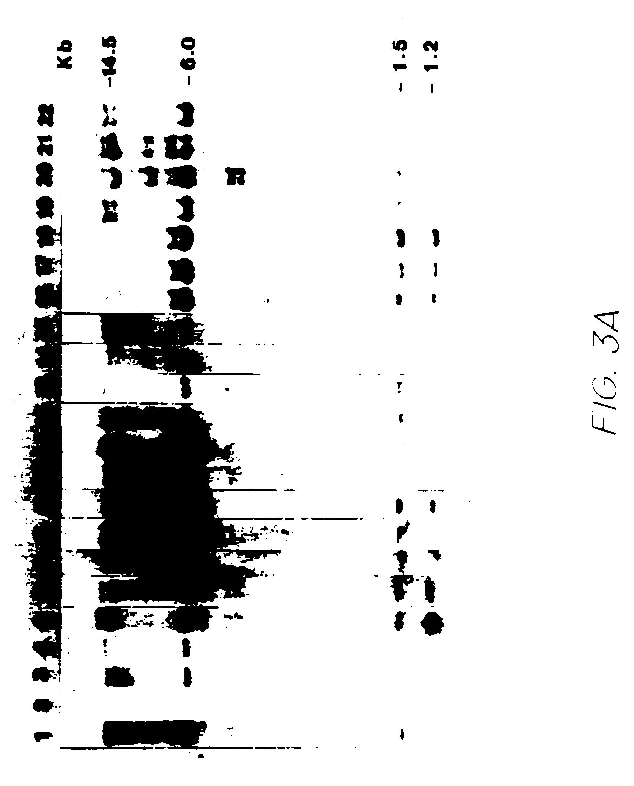

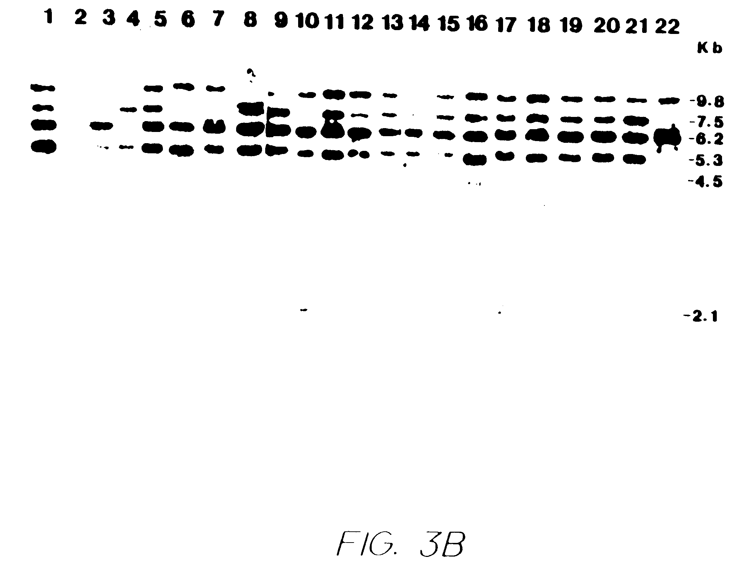

In both Figure 3A and 3B, Lane 1 is normal control human fibroblasts; 2, LA-RB128B (Tumor); 3, LA-RB128B-F (Fibroblast); 4, LA-RB165 (Tumor); 5, LA-RB165-F (Fibroblast); 6, LA-RB74 (Tumor); 7, LA-RB74-F (Fibroblast); 8, LA-RB151 (Tumor); 9, LA-RB151-F (Fibroblast); 10, OHS-50 (osteosarcoma); 11. LA-RB73 (Tumor); 12, LA-RB125 (Tumor); 13, LA-RB112 (Tumor); 14, LA-RB157 (Tumor); 15, OSV (osteosarcoma); 16, LA-RB94; (Tumor); 17, LA-RB70 (Tumor); 18, Y-79 (Tumor); 19, LA-RB69A (Tumor); 20, LA-RB69A-F (Fibroblast); 21, LA-RB99 (Tumor); 22, RB120F (Fibroblast that has a balanced translocation 46,XX,t [10, 13] [q22; q14])

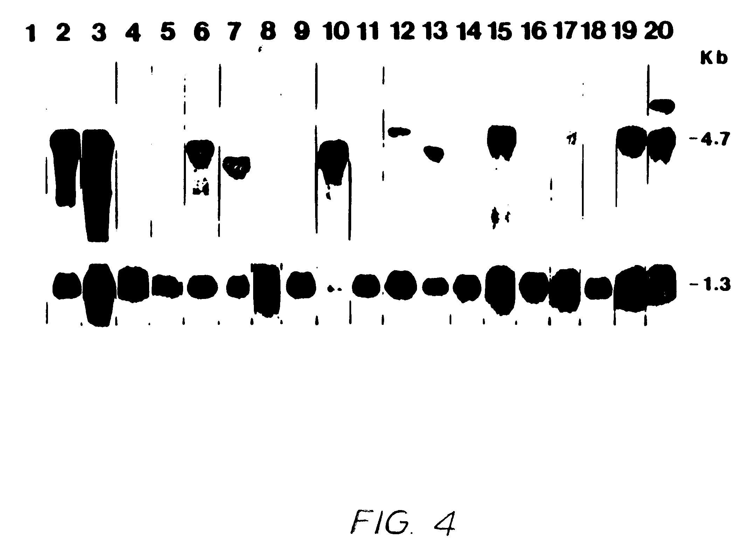

Figure 4 is a northern blot analysis of polyadenylated RNA from normal human fibroblast, normal and immortalized human retinal cells, retinoblastoma and their fibroblasts. Lane 1, Poly A-RNA; 2, Normal human fetal retinal cells; 3, Ad5/HER A (adenovirus 5 immortalized human retinal cells); 4, LA-RB128B; 5, LA-RB165; 6, LA-RB74; 7, OHS-50; 8, LA-RB73; 9, LA-RB157; 10, OSV; 11, LA-RB94; 12, LA-RB70; 13, Y-79, 14, LA-RB69A; 15, LA-RB69A-F (Fibroblast); 16, LA-RB133; 17, LA-RB110; 18, LA-RB160; 19, WS 1 (Fibroblast); 20, LA-RB99.

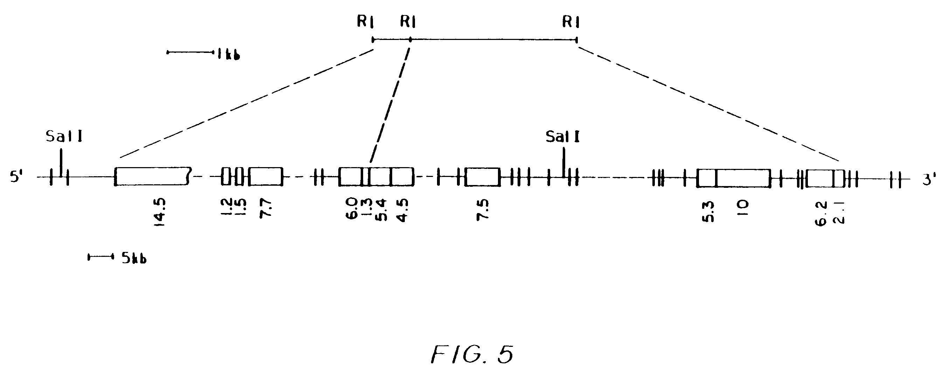

Figure 5 shows the Hind III restriction map of the Rb genomic locus.

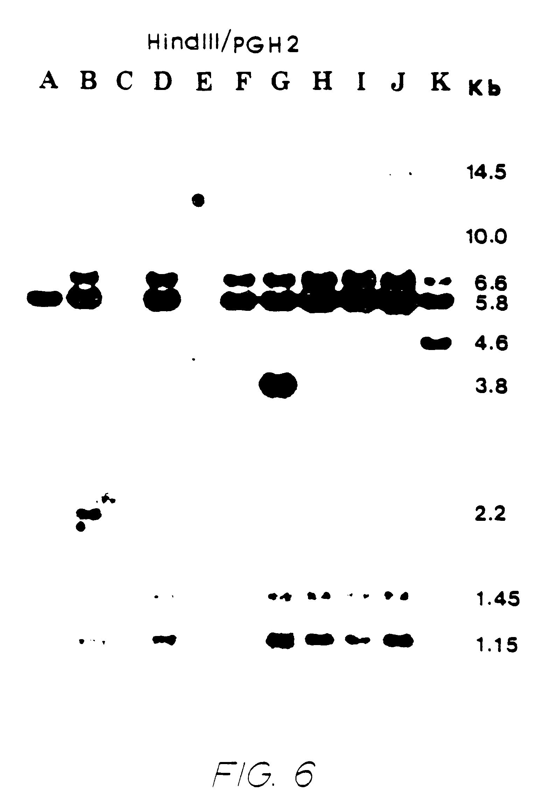

Figure 6 Southern Blot analysis of Hind III digested genomic DNA from human mammary tumor Lanes A, BT-549 (Infiltrating ductal carcinoma, human breast tumors); B, T-47D (Breast, duct carcinoma, pleural effusion); C, DU4475 (Metastiatic cutaneous nodule, breast--Human); D, MDA-MB-175-VII (Breast, ductal carcinoma, human); E, MDA-MB-468 (Human, breast, adenocarcinoma); F, HS0578T (ductal carcinoma, breast, human); G, ZR-5-30 (Breast carcinoma, human); H, BT-483 (Ductal carcinoma, breast, human); I, MDA-MB-415 (Adenocarcinoma, breast, human); J, MDA-MB-453 (Breast carcinoma, human); K, MDA-MB157 (breast carcinoma, human).

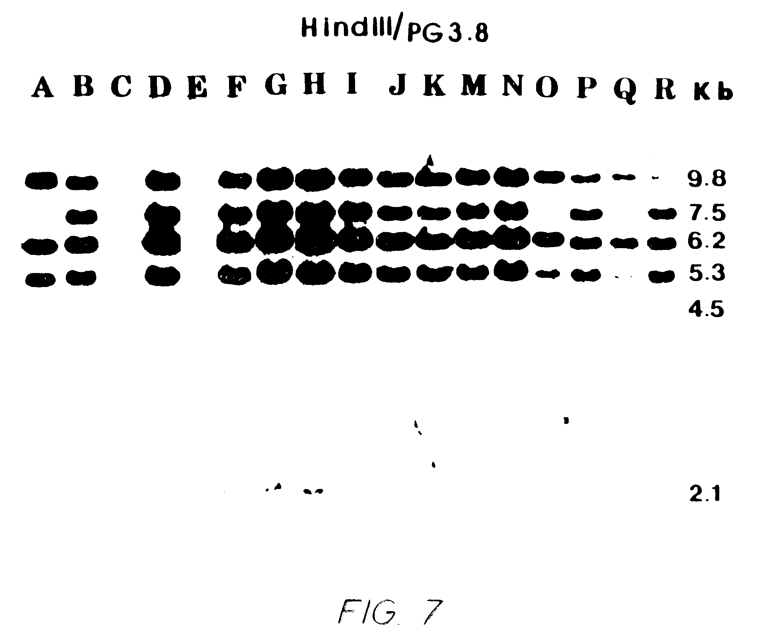

Figure 7. Southern Blot analysis of Hind III digested genomic DNA from human mammary tumors and a fibrosarcoma. Lanes A-K are the same as Fig. 6 and lanes L, MRMDA 231; M, SW613; N, MCF-7 (Breast, carcinoma, human); O, HS913T (fibrosarcoma); P, MDAMB 461; Q, MDAMB 436.

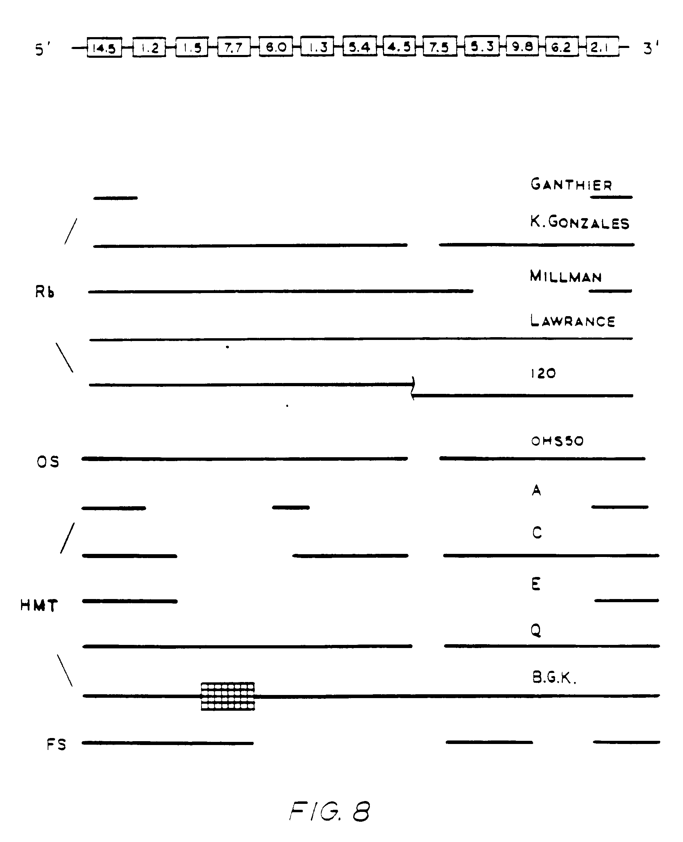

Figure 8 is a schematic representation of the summary of the structural aberrations detected in four human tumors. Rb = retinoblastoma, OS = osteosarcoma, HMT = human mammary tumor and FS = fibrosarcoma.

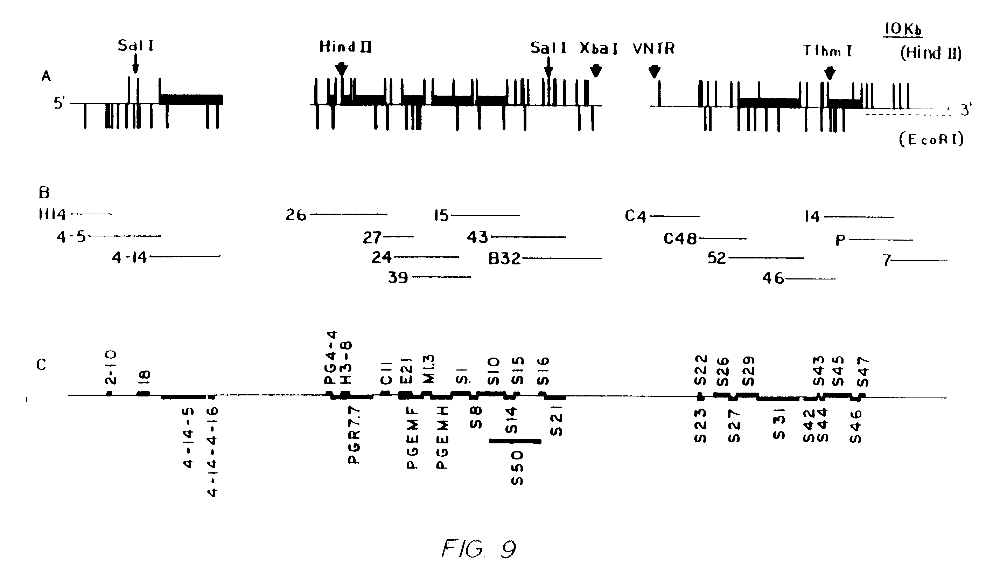

Figure 9A. Schematic representation of the retinoblastoma gene genomic locus. The restriction enzyme site for Hind III is shown above and those of EcoR1 shown below the line 5'3'. Shaded boxes represent exon-containing Hind III fragments. Bold arrow pointing to enzyme sites or areas which show restriction fragment length polymorphisms. VNTR, Variable number tandem repeats. Dotted lines represent regions that are not fully characterized.

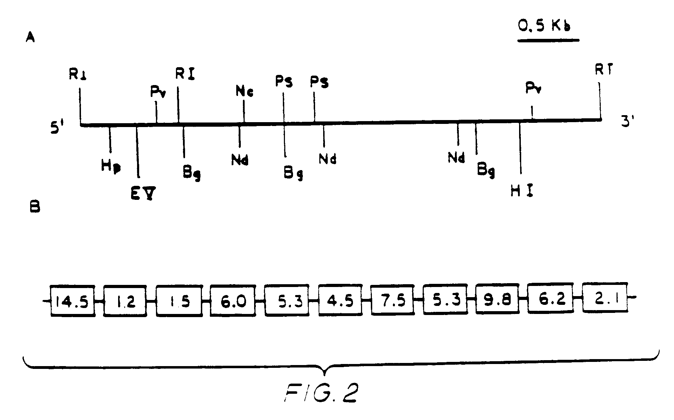

Figure 9B. Overlapping bacterial phage clones from which the restriction enzyme map in Figure 2.A. is deduced.

Figure 9C. Position and sizes of the various subclones of the Rb genomic locus DNA fragments from the bacterial phage clones were subcloned into PGEM plasmid vectors.

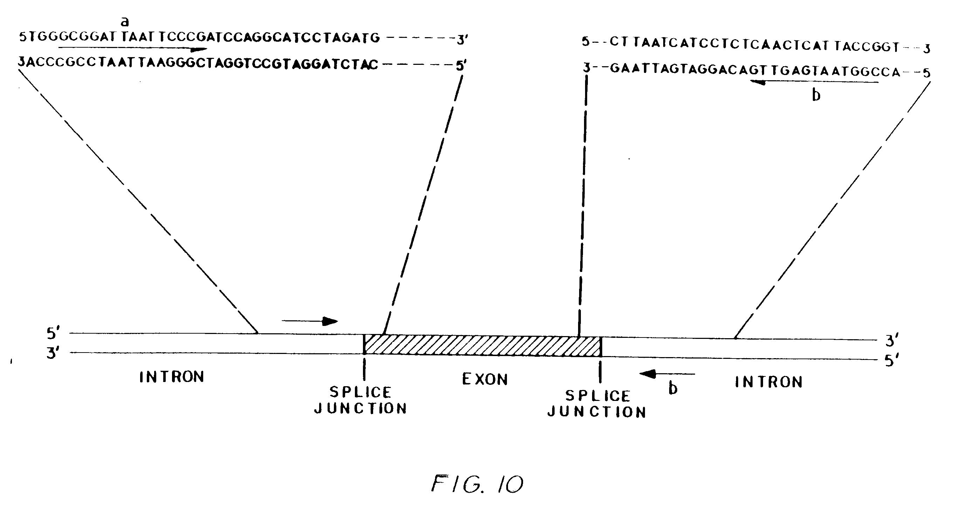

Figure 10. A hypothetical portion of the Rb genomic locus. The shaded area represent exons. Part of the nucleotide sequences covering the exon-intron junctions are shown above and below. For diagnosis purpose, a pair of DNA oligonucleotides a and b will be synthesized according to the 5'-3' sequences in the intron.

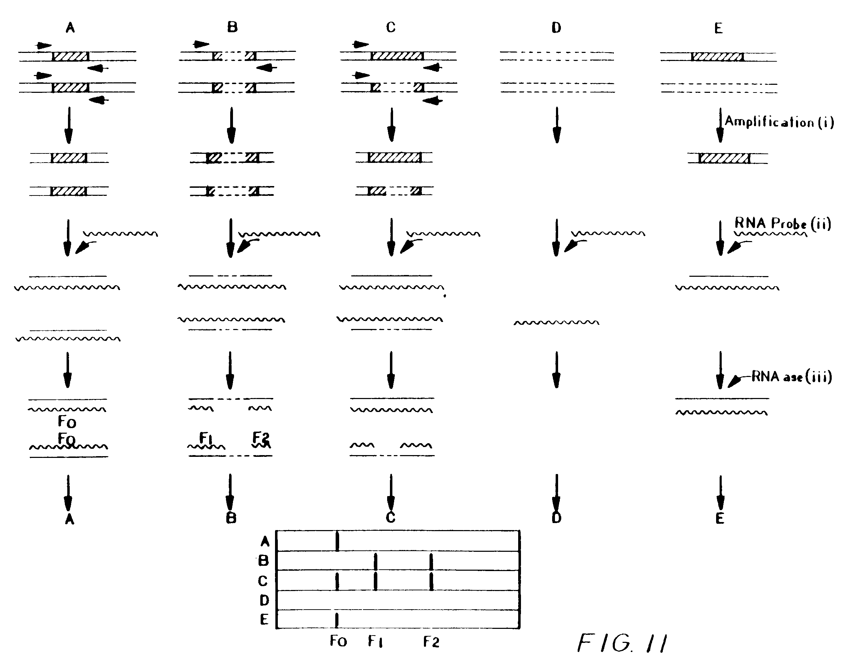

Figure 11. Application of RNAse protection assay for the diagnosis of mutations in a hypothetical region of the Rb genone. (i) the DNA from a normal person or from patient tissue is amplified using the pair of primers a and b which flank the exon; (ii) the amplified DNA is denatured and mixed with radioactively labelled ribonucleotide probe which in this case is longer than the amplified DNA; (iii) the mixed nucleic acids are allowed to anneal and are then digested with RNAase A. Single stranded portion of the RNA probe is digested but not the double stranded RNA-DNA hybrid; (iv) the double stranded hybrid is denatured into single stranded RNA and DNA which are then separated by electrophoresis in a gel; (v) the gel is exposed to X-ray film to reveal the size of the RNA molecule. Sources of DNA are as follows. A, Normal person; B, Tumor tissue that has a deletion or single base mutation inside the region flanked by oligonucleotide primer a and b; C, Constitutional cells from patient in B; D, Tumor tissue that has a deletion encompassing a and/or b; E Constitutional cells from patient in D.

The drawings are not necessarily to scale and certain features of the invention may be exaggerated in scale or shown in schematic form in the interest of clarity and conciseness.

DETAILED DESCRIPTION

A variety of tumors have been shown to have a structural defect in the retinoblastoma gene. This has been seen in tumor disorders such as fibrosarcoma, soft tissue sarcoma, small-cell lung cacinoma, melanoma cell lines and mammary tumor. The tumors containing the retinoblastoma gene defect can be detected by a method comprising the steps of: hybridizing a retinoblastoma gene probe to DNA extracted from a target tissue; and by determining the binding of the probe to said DNA. The binding can be measured by a variety of methods, including determining the amount of probe which hybridizes, determining structural changes, restriction fragment length polymorphism and amplifying the bound probe by the PCR method and measuring the product.

In one preferred embodiment, the probe is labelled and the hybridization is measured by determining the amount of label in the hybridized molecule. One skilled in the art will readily recognize that a variety of molecular biological methods are available to measure the hybridization itself or the product of the hybridization.

The target tissue is the sample which is obtained from individuals to be detected and from which the DNA is extracted. There is no limit on the type of tissue which may be collected and analyzed. For example, the tissues can be tumor, blood, fibroblasts, skin or other normal tissues, and can be taken from fetal to adult tissue.

One skilled in the art will readily recognize that a variety of labelled markers can be used with any of the retinblastoma probes. These markers can be radioactive, fluorescent or enzymatic. Furthermore, one skilled in the art will also recognize that other methods not employing a labelled probe can be used to determine the hybridization. For example, examination of restriction fragment length polymorphisms, amplification by the PCR method, RNAase A clevage and the allele specific oligonuleotide probing method.

# EP 0 364 490 B1

## DESCRIPTION OF Rb LOCUS

The Rb genomic locus is at least 200 kb long. A restriction map of the genomic locus is shown in Figure 9. The dotted lines represent regions that are not fully characterized. The remaining part of the genomic locus was isolated from human genomic libraries constructed in the bacterialphages λ EMBL3 and λ fix. The relationship of these phage clones to the genomic locus is shown in Figure 9. There are at least 20 exons in the genomic locus. These exons are located in 12 Hind III restriction fragments as shown in Figure 9. Restriction fragments containing the exons have been subcloned into the plasmid vector PGEM4 and PGEM2. The positions of the Hind III sites are shown above the line 5'-3' and the Eco R1 sites are shown below it. The shaded boxes represent the exon-containing Hind III DNA fragments. The fragments are included in the 4.6 kb cDNA probe for the retinoblastoma gene. Hybridization of Hind III or Eco R1 digested DNA to a labelled Rb cDNA clone reveals the various Hind III DNA fragments represented by the shaded boxes. Gross structural changes involving these fragments are detected.

Ribonuclease A (RNAase A) Cleavage Method - A small deletion involving very few nucleotides and a mutation at a single base will usually not be detectable by Southern blot analysis. These changes can be detected by the ribonuclease A (RNAase A) cleavage assay.

Since structural defects in the exons of the Rb genomic locus are the most likely causes of inactivation, initial diagnosis is focused on these regions. As shown in Figure 9, each of the shaded boxes represents an exon-containing Hind III DNA fragment. There are one or more exons in each of these fragments. The total number of exons is likely to exceed twenty.

In order to apply the RNAase A cleavage assay, pairs of DNA oligonucleotides are synthesized, Fig. 10. These pairs can be either parts of the exon or from the flanking region around each exon. To analyze a DNA sample for a mutation in an exon, a small amount of the DNA sample is mixed with the two oligonucleotides a and b. The mixture is subjected to polymerase chain reaction (PCR) using the GeneAmp ™ DNA amplification system from Perkin-Elmer Cetus. This method allows for selective amplification of the DNA flanked by the two oligonucleotides. The amplified DNA is denatured and then hybridized to a single strand radioactively labelled ribonucleotide synthesized from a subclone of the Rb genomic locus containing this region. The hybridized material is then subjected to digestion with RNAase A. A reaction consisting of DNA amplified from a normal person is run as a control. The digested product is fractionated by gel electrophoresis and the size of the radioactive ribonucleotide detected by exposure to X-ray film. The nucleotide sequence of the radioactive RNA probe corresponding to the amplified DNA from a normal person is protected from being digested by RNAase A. The size of the protected RNA fragment $F_O$, figure 11, is used as the standard.

For tumor DNA samples, deletions occuring within the region flanked by the two oligonucleotides can result in digestion of the hybrid into two smaller fragments $F_1$ and $F_2$, figure 11B. In the constitutional cells, the two fragments can also be detected if the mutation has occurred in one of the Rb alleles figure 11C. For tumor DNA samples with deletions encompassing one or both of the oligonucleotides a and b, no DNA amplification is possible. Therefore the RNA probe is not protected figure 11D. When using constitutional cells, there may be a problem. In such cases, one will have to search for another pair of oligonucleotide primers which flank the deleted region. Once a mutation can be detected in the Rb gene by these methods, it is possible to apply the same detection procedure on the immediate relatives of the proband. In cases where a family history is known, the segregation of chromosomes containing the mutated allele can be identified by using the enzymes and probe in the Rb gene which can detect RFLPs.

Linkage Studies Using Restriction Fragment Length Polymorphisms (RFLPs) -There are at least four regions in the RB genomic locus which show frequent polymorphism with a given enzyme. They are Kpn 1 in region 1, and Hind III in region 2, TthIII 1 in region 4 and a number of enzymes in region 3. Region 3 is a VNTR (variable number tandem reports) region, the enzymes RSal seem to be particularly useful in detecting polymorphism. DNA fragments generated by these enzymes may show size polymorphism when hybridized to the DNA probe from the region indicated. The segregation of a given Rb allele, as defined by these RFLPs, can thus be compared to the segregation and incidence of retinoblastoma in a family.

## DESCRIPTION OF THE Rb cDNA AND GENEOMIC DNA CLONES

The basic strategy of cloning involves chromosomal walking using probes in region 13q14.1, the locus of the Rb gene. Random probes free of highly repetitive sequences were isolated from the chromosome 13 specific library, LL 13NS01. To identify those clones that belonged to the 13q14.1 region, two hamster-human hybrid cell lines (3B6 and 2D12) were used. Each of these cell lines contained a chromosome 13 with a deletion including the 13q14.1 region. Cell hybrid 3B6 was made using the cell strain 32T which has

6

a chromosomal deletion from 13q14.1 to 13q14.3 whereas 2D12 contains a 13 chromosome with a deletion from 13q14.11 to 13q22.3. Clones containing DNA inserts within chromosomal region 13q14.1 to 13q14.3 were identified by the absence of hybridization to DNA from 3B6 and 2D12 using the inserts as probes. Two other probes, H2-42 and H3-8 can be obtained by a similar strategy. In addition, since both the EsD and Rb loci are in 13q14.1, we also isolated a full length EsD cDNA clone to use as a probe.

Each of these probes localized to 13q14.1 was used as a starting point for walking bidirectionally. A large number of clones were isolated using these various probes. Interestingly, one of these probes, H3-8, was found to be missing from several phage and cosmid libraries, presumably because it is in a region that is easily deleted and thus it was used to detect shorter restriction fragments. Figure 1A shows a restriction map of this region obtained by overlapping clones of different restriction fragments. The insert size of each clone obtained was verified by hybridization of the clone to genomic blots.

Surprisingly, a probe, PG4-4 was isolated, which was located very near H3-8 and which hybridized to a hamster sequence. This sequence conservation between human and hamster suggested that PG4-4 contains the cloning sequence of a gene. Hybridization of PG4-4 to an mRNA blot of several immortalized human fetal retinal cell lines revealed a 4.6 kilobase (kb) mRNA, Fig. 1B. Using PG4-4 as a probe, several cDNA clones were isolated. The longest of which was 4.6 kb. Figure 2A shows a restriction map of this cDNA clone. The polarity of the clones as shown in the figure was determined by hybridization of strand-specific probes to mRNA blots. The exon sequence in PG4-4 was found to be located between the Hpal and Eco RV sites near the 5' end of the cDNA. The nucleotide sequence of an 4.6 kb probe is shown in Table 1.

EP 0 364 490 B1

## Table 1.

5'

```
           10           20           30           40
CCCCCCAAAA   ACGGCCGCCA   CCGCCGCCGC   TGCCGCCGCG
           50           60           70           80
GAACCCCCGG   CACCGCCGCC   GCCGCCCCCT   CCTGAGGAGA
           90          100          110          120
ACCCAGAGCA   GGACAGCGGC   CCGGAGGACC   TGCCTCTCGT
          130          140          150          160
CAGGCTTGAG   TTTGAAGAAA   CAGAAGAACC   TGATTTTACT
          170          180          190          200
GCATTATGTC   AGAAATTAAA   GATACCAGAT   CATGTCAGAG
          210          220          230          240
AGAGAGCTTG   GTTAACTTGG   GAGAAAGTTT   CATCTGTGGA
          250          260          270          280
TGGAGTATTG   GGAGGTTATA   TTCAAAGAA    AAAGGAACTG
          290          300          310          320
TGGGGAATCT   GTATCTTTAT   TGCAGCAGTT   GACCTAGATG
          330          340          350          360
AGATGTCGTT   CACTTTTACT   GTGCTACAGA   AAAACATAGA
          370          380          390          400
AATCAGTGTC   CATAAATTCT   TTAACTTACT   AAAAGAAATT
          410          420          430          440
GATACCAGTA   CCAAAGTTGA   TAATGCTATG   TCAAGACTGT
          450          460          470          480
TGAAGAAGTA   TGATGTATTG   TTTGCACTCT   TCAGCAAATT
          490          500          510          520
GGAAAGGACA   TGTGAACTTA   TATATTTGAC   ACAACCCAGC
          530          540          550          560
AGTTCGATAT   CTACTGAAAT   AAATTCTGCA   TTGGTGCTAA
          570          580          590          600
AAGTTTCTTG   GATCACTTTT   TTATTAGCTA   AAGGGGAAGT
          610          620          630          640
ATTACAAATG   GAAGATGATC   TGGTGATTTC   ATTTCAGTTA
          650          660          670          680
ATGCTATGTG   TCCTTGACTA   TTTTATTAAA   CTCTCACCTC
          690          700          710          720
CCATGTTGCT   CACCCAGGCG   TATAAAACAG   CTGTTATACC
          730          740          750          760
CATTAATGGT   TCACCTCGAA   AACCCCAGGC   GGGTCAGAAC
          770          780          790          800
AGGAGTGCAC   GGATAGCAAA   ACAACTAGAA   AATGATACAA
          810          820          830          840
GAATTATTGA   AATTCTCTGT   AAAGAACATG   AATGTAATAT
          850          860          870          880
AGATGAGGTG   AAAAATGTTT   ATTTCAAAAA   TTTTATACCT
          890          900          910          920
TTTATGAATT   CTCTTGGACT   TGTAACATCT   AATGGACTTC
          930          940          950          960
CAGCGGTTGA   AAATCTTTCT   AAACGATACG   AAGAAATTTA
```

8

|       970 |       980 |       990 |      1000 |
|-----------|-----------|-----------|-----------|
| TCTTAAAAAT | AAAGATCTAG | ATGCAAGATT | ATTTTTGCAT |
|      1010 |      1020 |      1030 |      1040 |
| CATGATAAAA | CTCTTCAGAC | TGATTCTATA | GACAGTTTTG |
|      1050 |      1060 |      1070 |      1080 |
| AAACACAGAG | AACACCACGA | AAAAGTAACC | TTGATGAAGA |
|      1090 |      1100 |      1110 |      1120 |
| GGTGAATGTA | ATTCCTCCAC | ACACTCCAGT | TAGGACTGTT |
|      1130 |      1140 |      1150 |      1160 |
| ATGAACACTA | TCCAACAATT | AATGATGATT | TTAAATTCAG |
|      1170 |      1180 |      1190 |      1200 |
| CAAGTGATCA | ACCTTCAGAA | AATCTGATTT | CCTATTTTAA |
|      1210 |      1220 |      1230 |      1240 |
| CAACTGCACA | GTGAATCCAA | AAGAAAGTAT | ACTGAAAAGA |
|      1250 |      1260 |      1270 |      1280 |
| GTGAAGGATA | TAGGATACAT | CTTTAAAGAG | AAATTTGCTA |
|      1290 |      1300 |      1310 |      1320 |
| AAGCTGTGGA | CAGGGTTGTG | TCGAAATTGG | ATCACAGCGA |
|      1330 |      1340 |      1350 |      1360 |
| TACAAACTTG | GAGTTCGCTT | GTATTACCGA | GTAATGGAAT |
|      1370 |      1380 |      1390 |      1400 |
| CCATGCTTAA | ATCAGAAGAA | GAACGATTAT | CCATTCAAAA |
|      1410 |      1420 |      1430 |      1440 |
| TTTTAGCAAA | CTTCTGAATG | ACAACATTTT | TCATATGTCT |
|      1450 |      1460 |      1470 |      1480 |
| TTATTGGCGT | GCGCTCTTGA | GGTTGTAATG | GCCACATATA |
|      1490 |      1500 |      1510 |      1520 |
| GCAGAAGTAC | ATCTCAGAAT | CTTGATTCAG | GAACAGATTT |
|      1530 |      1540 |      1550 |      1560 |
| GTCTTTCCCA | TGGATTCTGA | ATGTGCTTAA | TTTAAAAGCC |
|      1570 |      1580 |      1590 |      1600 |
| TTTGATTTTT | ACAAAGTGAT | CGAAAGTTTT | ATCAAAGCAG |
|      1610 |      1620 |      1630 |      1640 |
| AAGGCAACTT | GACAAGAGAA | ATGATAAAAC | ATTTAGAACG |
|      1650 |      1660 |      1670 |      1680 |
| ATGTGAACAT | CGAATCATGG | AATCCTTTGC | ATGGCTCTCA |
|      1690 |      1700 |      1710 |      1720 |
| GATTCACCTT | TATTTGATCT | TATTAAACAA | TCAAAGGACC |
|      1730 |      1740 |      1750 |      1760 |
| GAGAAGGACC | AACTGATCAC | CTTGAATCTG | CTTGTCCTCT |
|      1770 |      1780 |      1790 |      1800 |
| TAATCTTCCT | CTCCAGAATA | ATCACACTGC | AGCAGATATG |
|      1810 |      1820 |      1830 |      1840 |
| TATCTTTCTC | CTGTAAGATC | TCCAAAGAAA | AAAGGTTCAA |
|      1850 |      1860 |      1870 |      1880 |
| CTACGCGTGT | AAATTCTACT | GCAAATGCAG | AGACACAAGC |
|      1890 |      1900 |      1910 |      1920 |
| AACCTCAGCC | TTCCAGACCC | AGAAGCCATT | GAAATCTACC |
|      1930 |      1940 |      1950 |      1960 |
| TCTCTTTCAC | TGTTTTATAA | AAAAGTGTAT | CGGCTAGCCT |
|      1970 |      1980 |      1990 |      2000 |
| ATCTCCGGCT | AAATACACTT | TGTGAACGCC | TTCTGTCTGA |

```
        2010         2020         2030         2040
   GCACCCAGAA   TTAGAACATA   TCATCTGGAC   CCTTTTCCAG
        2050         2060         2070         2080
   CACACCCTGC   AGAATGAGTA   TGAACTCATG   AGAGACAGGC
        2090         2100         2110         2120
   ATTTGGACCA   AATTATGATG   TGTTCCATGT   ATGGCATATG
        2130         2140         2150         2160
   CAAAGTGAAG   AATATAGACC   TTAAATTCAA   AATCATTGTA
        2170         2180         2190         2200
   ACAGCATACA   AGGATCTTCC   TCATGCTGTT   CAGGAGACAT
        2210         2220         2230         2240
   TCAAACGTGT   TTTGATCAAA   GAAGAGGAGT   ATGATTCTAT
        2250         2260         2270         2280
   TATAGTGTTC   TATAACTCGG   TCTTCATGCA   GAGACTGAAA
        2290         2300         2310         2320
   ACAAATATTT   TGCAGTATGC   TTCCACCAGG   CCCCCTACCT
        2330         2340         2350         2360
   TGCACCAATA   CCTCACATTC   CTCGAAGCCC   TTACAAGTTT
        2370         2380         2390         2400
   CCTAGTTCAC   CCTTACGGAT   TCCTGGAGGG   AACATCTATA
        2410         2420         2430         2440
   TTTCACCCCT   GAAGAGTCCA   TATAAAATTT   CAGAAGGTCT
        2450         2460         2470         2480
   GCCAACACCA   ACAAAAATGA   CTCCAAGATC   AAGAATCTTA
        2490         2500         2510         2520
   GTATCAATTG   GTGAATCATT   CGGGACTTCT   GAGAAGTTCC
        2530         2540         2550         2560
   AGAAAATAAA   TCAGATGGTA   TGTAACAGCG   ACCGTGTGCT
        2570         2580         2590         2600
   CTAAAGAAGT   GCTGAAGGAA   GCAACCCTCC   TAAACCACTG
        2610         2620         2630         2640
   AAAAAACTAC   GCTTTGATAT   TGAAGGATCA   GATGAAGCAG
        2650         2660         2670         2680
   ATGGAAGTAA   ACATCTCCCA   GGAGAGTCCA   AATTTCAGCA
        2690         2700         2710         2720
   GAAACTGGCA   GAAATGACTT   CTACTCGAAC   ACGAATGCAA
        2730         2740         2750         2760
   AAGCAGAAAA   TGAATGATAG   CATGGATACC   TCAAACAAGG
        2770         2780         2790         2800
   AAGAGAAATG   AGGATCTCAG   GACCTTGGTG   GACACTGTGT
        2810         2820         2830         2840
   GCACCTCTGG   ATTCATTGTC   TCTCACAGAT   GTGACTGTAT
        2850         2860         2870         2880
   AACTTTCCCA   GGTTCTGTTT   ATGGCCACAT   TTAATATCTT
        2890         2900         2910         2920
   CAGCTCTTTT   TGTGGATATA   AAATGTGCAG   ATGCAATTGT
        2930         2940         2950         2960
   TTGGGTGATT   CCTAAGCCAC   TTGAAATGTT   AGTCATTGTT
        2970         2980         2990         3000
   ATTTATACAA   GATTGAAAAT   CTTGTGTAAA   TCCTGCCATT
        3010         3020         3030         3040
   TAAAAAGTTG   TAGCAGATTG   TTTCCTCTTC   CAAAGTAAAA
```

10

```
         3050            3060            3070            3080
    TTGCTGTGCT      TTATGGATAG      TAAGAATGGC      CCTAGAGTGG
         3090            3100            3110            3120
    GAGTCCTGAT      AACCCAGGCC      TGTCTGACTA      CTTTGCCTTC
         3130            3140            3150            3160
    TTTTGTAGCA      TATAGGTGAT      GTTTGCTCTT      GTTTTTATTA
         3170            3180            3190            3200
    ATTTATATGT      ATATTTTTT       AATTTAACAT      GAACACCCTT
         3210            3220            3230            3240
    AGAAAATGTG      TCCTATCTAT      CTTCCAAATG      GAATTTGATT
         3250            3260            3270            3280
    GACTGCCCAT      TCACCAAAAT      TATCCTGAAC      TCTTCTGCAA
         3290            3300            3310            3320
    AAATGGATAT      TATTAGAAAT      TAGAAAAAAA      TTACTAATTT
         3330            3340            3350            3360
    TACACATTAG      ATTTTATTTT      ACTATTGGAA      TCTGATATAC
         3370            3380            3390            3400
    TGTGTGCTTG      TTTTATAAAA      TTTTGCTTTT      AATTAAATAA
         3410            3420            3430            3440
    AAGCTGGAAG      CAAAGTATAA      CCATATGATA      CTATCATACT
         3450            3460            3470            3480
    ACTGAAACAG      ATTTCATACC      TCAGAATGTA      AAAGAACTTA
         3490            3500            3510            3520
    CTGATTATTT      TCTTCATCCA      ACTTATGTTT      TTAAATGAGG
         3530            3540            3550            3560
    ATTATTGATA      GTACTCTTGG      TTTTTATACC      ATTCAGATCA
         3570            3580            3590            3600
    CTGAATTTAT      AAAGTACCCA      TCTAGTACTT      GAAAAGTAA
         3610            3620            3630            3640
    AGTGTTCTGC      CAGATCTTAG      GTATAGAGGA      CCCTAACACA
         3650            3660            3670            3680
    GTATATCCCA      AGTGCACTTT      CTAATGTTTC      TGGGTCCTGA
         3690            3700            3710            3720
    AGAATTAAGA      TACAAATTAA      TTTTACTCCA      TAAACAGACT
         3730            3740            3750            3760
    GTTAATTATA      GGAGCCTTAA      TTTTTTTTTC      ATAGAGATTT
         3770            3780            3790            3800
    GTCTAATTGC      ATCTCAAAAT      TATTCTGCCC      TCCTTAATTT
         3810            3820            3830            3840
    GGGAAGGTTT      GTGTTTCTC       TGGAATGGTA      CATGTCTTCC
         3850            3860            3870            3880
    ATGTATCTTT      TGAACTGGCA      ATTGTCTATT      TATCTTTTAT
         3890            3900            3910            3920
    TTTTTAAGT       CAGTATGGTC      TAACACTGGC      ATGTTCAAAG
         3930            3940            3950            3960
    CCACATTATT      TCTAGTCCAA      AATTACAAGT      AATCAAGGGT
         3970            3980            3990            4000
    CATTATGGGT      TAGGCATTAA      TGTTTCTATC      TGATTTTGTG
         4010            4020            4030            4040
    CAAAAGCTTC      AAATTAAAAC      AGCTGCATTA      GAAAAGAGG
         4050            4060            4070            4080
    CGCTTCTCCC      CTCCCCTACA      CCTAAAGGTG      TATTTAAACT
```

```
         4090              4100              4110              4120
    ATCTGTGTGA        TTAACTTATT        TAGAGATGCT        GTAACTTAAA
         4130              4140              4150              4160
    ATAGGGGATA        TTTAAGGTAG        CTTCAGCTAG        CTTTTAGGAA
         4170              4180              4190              4200
    AATCACTTTG        CCTAACTCAG        AATTATTTTT        AAAAAGAAAT
         4210              4220              4230              4240
    CTGGTCTTGT        TAGAAAACAA        AATTTTATTT        TGTGCTCAGT
         4250              4260              4270              4280
    TAAGTTTCAA        ACTTACTATT        TTGACAGTTA        TTTTGATAAC
         4290              4300              4310              4320
    AATGACACTA        GAAAACTTGA        CTCCATTTCA        TCATTGTTTC
         4330              4340              4350              4360
    TGCATGAATA        TCATACAAAT        CAGTTAGTTT        TTAGGTCAAG
         4370              4380              4390              4400
    GCCTTACTAT        TTCTGGGTCT        TTTGCTACTA        AGTTCACATT
         4410              4420              4430              4440
    AGAATTAGTG        CCAGAATTTT        AGGAACTTCA        GAGATCGTGT
         4450              4460              4470              4480
    ATTGAGATTT        CTTAAATAAT        GCTTCAGATA        TTATTGCTTT
         4490              4500              4510              4520
    ATTGCTTTTT        TGTATTGGTT        AAAACTGTAC        ATTTAAAATT
         4530              4540              4550              4560
    GCTATGTTAC        TATTTCTAC         AATTAATAGT        TTGTCTATTT
         4570              4580
    TAAAATAAAT        TAGTTGTTAG        3'
```

This cDNA probe is comprised of at least 20 exons. Thus, multiple smaller probes (fragments) can be made from this large probe. Furthermore, one skilled in the art will recognize that derivatives of this probe, for example, degenerate sequences or sequences with a few single base pair changes will also work as effective probes.

Part of the 5' end of the Rb gene is missing from the genomic clone. This part which included the promoter and part of the first exon is shown in Table 2.

Table 2.

```
5'
                 10              20              30              40
            CCGCGAGGTG      ATTAGCAGAT      TATTCCTCTG      CGGGGCCCGA
                 50              60              70              80
            GAGTCTTCCC      TATCAGACCC      CGGGATAGGG      ATGAGGCCCA
                 90             100             110             120
            GCAGTCACCC      ACCAGACTCT      TTGTATAGCC      CGTTAAGTGG
                130             140             150             160
            ACCCGGCCGT      GAGGGGGTGG      TTCTGGGTAA      AAGACGTCCG
                170             180             190             200
            GGCCGCGCCG      GATGCCTCCT      GGAAGGCGCC      TGGACCCACG
                210             220             230             240
            CCAGGTTTCC      CACTTTAATT      CCTCATGACT      TAGCGTCCCA
                250             260             270             280
            GCCCGCGCAC      CGACCAGCGC      CCCAGTTCCC      CACAGACGCC
                290             300             310             320
            GGCGGGCCCG      GGAGCCTCGG      ACGTGAGCGG      CGGGCGGAAG
                330             340             350             360
            TGACGTTTTC      CCGCGGTTGG      ACGCGGCGCT      CAGTTGCCGG
                370             380             390             400
            GCGGGGGAGG      GCGCGTCCGG      TTTTTCTCAG      GGGACGTTGA
                410             420             430             440
            AATTATTTTT      GTAACGGGAG      TCGGGAGAGG      ACGGGCGTG
                450             460             470             480
            CCCCGACGTG      CGCGCGCGCG      TCCTCCCCGG      CGCTCCTCAC
                490             500             510             520
            AGCCTTCGCT      CGGTCCCCGG      CCGCGGAACC      GGTATGCCGC
                526
            CCAAAA          3'
```

In the genomic clone Table 1 and 2 are continuous such that Base 1 of Table 1 would be base 527 of Table 2.

This data shows a GC rich region between the extreme 5' end and the Hpal site. Hybridization of Southern blots to a probe made from this GC rich region invariably gave a smeared pattern.

The Rb available probes provide a means to identify whether or not there are structural changes in the Rb gene in a given tumor type or subset within a given tumor type. Therefore, it is possible that certain tumors will respond differently to therapy based on whether or not there is a defect in the Rb gene. Also since it is likely that functional loss of the Rb gene is critical to tumor development it may be possible to treat these tumors by the addition of the gene or gene product back into the cells.

Southern blot analysis - DNA is extracted from target tissues for example, tumor, blood, fibroblast or other normal tissues. The DNA is separately disgested with different restriction enzymes. The restriction enzyme fragments are separated in an agarose gel by electrophoresis, are denatured and then transferred to a nitrocellulose filter. Labelled cDNA probes of the Rb gene are hybridized to the DNA fragment on the filter. In the preferred embodiment the label is radioactive and the filter is exposed to X-ray films.

If the cDNA clone is the putative Rb gene, it should detect structural anomalies in the DNA of certain retinoblastomas, including those with visible deletions in the 13q14.1 region. To analyze the structure of genomic DNA from retinoblastomas, a restriction endonucleases survey was used to find enzymes which would generate well separated restriction fragments detectable with a cDNA probe. Hind III generated well-separated restriction fragments in normal human DNA that could be detected by subclones of the 4.73 kb cDNA clone. The order of the genomic Hind III restriction fragments detectable with a cDNA probe is shown in Fig. 2B.

Since a cDNA probe was used, only structural aberrations of the exon-containing restriction fragments will be detectable. Structural aberrations in restriction fragments containing a very small exon may not be detected. This is also true for point mutations or extremely small deletions in the exon or in splicing junctions. However, even with these limitations structural aberrations are observed, including part or all of the Rb gene. These various forms of structural changes are summarized in Table 3.

Table 3

| Tumor | Form of Disease | Structural Change in Rb Gene | Expression of Rb Gene at mRNA Level |
|---|---|---|---|
| LA-RB128B | Deletion | Homozygous Total Deletion | Absent |
| LA-RB165 | Unilateral | Homozygous 3' Deletion | Absent |
| LA-RB74 | Bilateral | Homozygous Internal Deletion | Abnormal |
| LA-RB151 | Bilateral | Homozygous Internal Deletion | ND+ |
| OHS-50 | Bilateral* | Homozygous Internal Deletion | Abnormal |
| LA-RB125 | Unilateral | Internal Deletion of 1 Allele | ND |
| LA-RB99 | Unilateral | Internal Deletion of 1 Allele | Abnormal |
| LA-RB112 | Unilateral | Total Deletion of 1 Allele | ND |
| LA-RB157 | Bilateral | Total Deletion of 1 Allele | Absent |
| OSV | Primary Oste-sarcoma | Total Deletion of 1 Allele | Abnormal |
| LA-RB69A | Bilateral | None Detectable | Absent |
| LA-RB73 | Bilateral | None Detectable | Absent |
| LA-RB110 | Bilateral | None Detectable | Absent |
| LA-RB133 | Bilateral | None Detectable | Absent |
| LA-RB160 | Unilateral | None Detectable | Absent |
| LA-RB94 | Unilateral | None Detectable | Abnormal |
| LA-RB70 | Unilateral | None Detectable | Reduced |
| Y-79 | Bilateral | None Detectable | Abnormal |

\* Osteosarcoma in patient with bilateral retinoblastoma
+ Not determined

Example 1

DNA from forty cases of retinoblastoma patients and from fibroblasts of the same patient were examined by Southern blot analysis. To facilitate this analysis, the 0.9 kb and 3.8 kb Eco R1 fragments were subcloned into the vector PGEM-4, Fig. 2A. Southern blots of genomic DNA isolated from the retinoblastomas digested with different restriction endonucleases were hybridized to probes made from these two subclones, PG H2 (Eco R1 0.9 kb) and PG 3.8M (Eco R1 3.8 kb). Polyadenylated RNA from several retinoblastomas were also analyzed with these probes.

One of the mechanisms by which the two Rb alleles are inactivated is the total or partial deletion of both Rb alleles. An example of the total deletion of both alleles is provided by the tumor, LA-RB128B, from a patient with bilateral retinoblastoma. Cytogenetic analysis of fibroblasts from this individual using high resolution banding revealed a visible deletion in one 13 chromosome from 13q14.1 to 13q14.3. The remaining 13 chromosome appeared normal. Consistent with these observations, the intensities of the Rb restriction fragments indicated the presence of only a single copy of an apparently intact Rb locus in the

14

fibroblasts, Lane 3 in both Figures 3A and 3B. RFLP studies using chromosome 13 probes above and below the 13q14.1 region showed that both chromosome 13s were present in the tumor. However, both alleles of the Rb locus were missing, Fig. 3A and 3B, lane 2. Thus, in this patient both the first and second mutational event involved deletion of one Rb allele. As expected, no Rb transcripts could be detected in the tumor, Fig. 4, lane 4.

LA-RB165, represents an example of a partial deletion. This tumor was obtained from a patient with unilateral disease. As shown in Fig. 3B lane 4, the Hind III 9.8 kb, 6.2 kb, and 2.1 kb fragments are missing. The intensities of the remaining Rb fragments indicate the existence of only a single copy of the aberrant Rb locus. Thus, in this tumor one allele of the Rb gene is totally deleted and the other has a deletion at the 3' end. As can be seen in Fig. 3A and B lanes 5, two copies of apparently intact Rb loci are present in the fibroblasts. This implies two different mutational events occurred in the somatic cell (i.e., the target retinoblast), a result that is consistent with the unilateral (non-hereditary) status of this retinoblastoma patient. No Rb transcript was detected in this tumor, Fig. 4, lane 5.

While a correlation between gross structural deletions and inactivation of the Rb gene is consistent with the assignment of the Rb gene to this region, homozygous total deletion or common 3' deletions do not exclude the possibility that the Rb gene is downstream to this region. The definitive proof of the authenticity of the Rb gene would either be the existence of a 5' deletion or homozygous internal deletion in other tumors. Two retinoblastomas and one osteosarcoma indeed were found to have such deletions.

The first example, tumor LA-RB74, came from a patient with bilateral retinoblastoma. As shown in Fig. 3B, lane 6, the 7.5 kb a Hind III restriction fragment is missing. One copy of a new 4.1 kb band could be detected. In this tumor, the Rb transcript is reduced in size to 4.4 kb, consistent with the deletion of an exon present in the missing 7.5 kb Hind III fragment, Fig. 4, lane 6. The restriction pattern of the fibroblasts from the same patient is shown in Fig. 3B, lane 7. The presence of one copy each of the normal 7.5 and the aberrant 4.1 kb Hind III fragment indicates that this patient has a germline deletion in this region.

A second example of a homozygous internal deletion is seen in tumor LA-RB151, also from a patient with bilateral retinoblastoma. RFLP studies showed that chromosome 13 probes both above and below the Rb locus were heterozygous in fibroblast cells and became homozygous in tumor cells. In the tumor both copies of the 9.8 kb Hind III were missing and two copies of a novel 8.0 kb Hind III fragment were present, Fig. 3B, lane 8. Judging from the intensity of the 9.8 kb Hind III fragment in the fibroblasts, Fig. 3B, lane 9, we conclude that only a single copy of the 9.8 kb Hind III fragment was present in the fibroblasts.

A third example of a homozygous internal deletion was found in the osteosarcoma, OHS. This tumor was obtained from a patient who had a previous history of retinoblastoma. As can be seen from Fig. 3B, lane 10, the 7.5 kb Hind III fragment is missing. The small amount of background hybridization to the 7.5 kb fragment presumably comes from a few contaminating normal cells. Interestingly, while both OHS and LA-RB74 have a deletion of the 7.5 kb Hind III fragment and truncated transcripts, the Rb gene transcript in OHS is reduced to 3.9 kb, Fig. 4, lane 7, which is smaller than that for LA-RB74, Fig. 4, lane 6.

Tumor LA-RB125 is from a patient with unilateral sporadic retinoblastoma. As shown in Fig. 3B, lane 12, only a single copy of the 7.5 kb Hind III fragment is present, in contrast to two copies of the rest of the Rb gene. Apparently one of the Rb alleles contains an internal deletion in the 7.5 kb region.

LA-RB99 is from a unilateral case of retinoblastoma in which an internal deletion involving the Hind III 9.8 kb fragment can be detected in one of the Rb alleles, Fig. 3B, lane 21. Instead, a new fragment also of 7.5 kb is generated. Two different size transcripts, a 7.0 kb and a normal sized 4.7 kb can be detected in the tumor, Fig. 4, lane 20. In both of these cases an abnormality could be detected in only one of the chromosome 13s.

Three tumors with total deletion of one of the Rb alleles are shown in lanes 13, 14 and 15 of Figs. 3A and 3B. LA-RB112, lane 13, was from a patient with unilateral sporadic disease, LA-RB157, lane 14, was from a bilateral case and OSV, lane 15, is an osteosarcoma from a patient with no known history of retinoblastoma. In all these cases, only a single, apparently normal Rb locus remains. Little or no Rb transcript can be detected in LA-RB157, Fig. 4, lane 9. In OSV, however, a truncated message of 4.2 kb can be detected, Fig. 4, lane 10.

The presence of a stretch of a GC rich region at the extreme 5' end of the cDNA complicated the analysis of the genomic locus. If we assume that the 14.5 kb Hind III fragment, Fig. 2B, contains the first exon of the Rb gene then several of the cases of retinoblastoma have 5' deletions. If, however, there exists yet another small exon in front of the 14.5 kb Hind III fragment then these cases would represent additional examples of internal deletions.

Approximately 60% of the forty retinoblastomas show no apparent structural changes in the Rb gene. Among this group of tumors, various abnormalities are observed in Rb transcript expression. For example, none of the following five tumors express a Rb transcript: the bilateral retinoblastomas LA-RB73, Fig. 4, lane 8; LA-RB69A, Fig. 4, lane 14; and LA-RB133, Fig. 4, lane 16, all of which are homozygous for a chromosome 13; the bilateral tumor LA-RB110, Fig. 4, lane 17, which is heterozygous for chromosome 13 polymorphic probes both above and below the Rb locus; and the unilateral tumor LA-RB160, Fig. 4, lane 18, which has become homozygous for a chromosome 13 probe, 7D2, that is also located in chromosomal region 13q14.

In other cases abnormal transcripts are also detected. For example, the unilateral case, LA-RB94 which is homozygous for chromosome 13, shows no gross structural changes, Fig. 3A and 3B, lane 16, but has a small amount of an apparently larger Rb transcript, Fig. 4, lane 11. This presumably is due to the loss of a splice junction such that a small intron is included in the final transcript. LA-RB70, which also has no apparent change in the Rb DNA locus, Fig. 3A and 3B, lane 17, has a reduced amount of a normal size Rb transcript, Fig. 4, lane 12. Finally, Y79, the most commonly used retinoblastoma cell line to date, has consistently shown a reduced amount of a truncated Rb transcript of 4.2 kb, Fig. 4B, lane 13, although no gross DNA changes can be observed with the present probes, Fig. 3A and 3B, lane 18. Presumably, a yet undetected deletion has occurred in the Rb locus.

A homozygous internal deletion and truncated Rb transcript were also observed in an osteosarcoma from a patient with a prior history of retinoblastoma. These data are consistent with the postulate that the Rb gene is responsible for the development of both retinoblastoma and osteosarcoma. The observation that the majority of the tumors do not express the Rb gene at the RNA level provide strong support for the recessive nature of the Rb gene and the contention that it is the loss of function of both Rb alleles which is responsible for tumor development.

In all the internal deletion cases examined, deletions always involved either the 7.5 or 9.8 kb Hind III fragments. One copy of the Hind III 7.5 kb fragment was also found to be altered in the RB120F fibroblasts, Fig. 3B lane 22. This fibroblasts cell line was from a patient with bilateral retinoblastoma in which a balanced translocation involving chromosomal region 13q14 and 10q22 had occurred. A novel band of 6.5 kb was detected instead. Whether these regions of the Rb gene locus contain peculiar structures that would predispose them to deletion or translocation remains to be examined.

This data, LA-RB128B, LA-RB74, and LA-RB151 also provided a verification of Knudson's two-hit hypothesis. In bilateral cases a mutation at the Rb locus representing the first hit was readily detected in the fibroblasts, Fig. 3B, lanes 3, 7, and 9, and the second hit was found in the tumor, Fig. 3B, lanes 2, 6 and 8. In the unilateral case, LA-RB165, the two mutational events, 3' deletion and total deletion, were seen only in the tumor, Fig. 3B, lane 4, and not in the fibroblasts, Fig. 3B, lane 5.

Example 2

It has been observed that the retinoblastoma gene is similarly inactivated in some human mammary tumors, human melanoma, small-cell carcinoma of the lung, soft tissue sarcoma and fibrosarcoma. These observations provide the basis for the detection of defects of the retinoblastoma gene in the cells of these patients.

The human mammary carcinoma cell lines used were obtained from American type culture collection. The techniques employed were as previously described for the retinoblastoma tumors.

The genomic Rb locus, Fig. 5, is depicted as exons containing Hind III and Sal 1 restriction fragments. The cDNA clones are divided into two portions for synthesis of probes. Figure 6 shows Southern blot analysis of the Hind III digested genomic DNA from eleven human mammary tumors. As can be seen, five of these tumors, A, C, E, G and K, show structural aberrations in this region. Figure 7 shows a Southern blot analysis of the Hind III digested genomic DNA from sixteen human breast tumor cell lines, including the eleven samples shown in Figure 6, and one fibrosarcoma cell line, lane O.

As can be seen from Figure 7, additional structural aberrations can be seen. In addition, one human breast tumor line, lane A, and the fibrosarcoma line, lane O, both show structural aberrations in this part of the Rb gene locus.

A summary of the structural changes is shown in Figure 8. The exon-containing Hind III fragments are shown on top while the structural changes are shown below. Internal deletions at the 7.5 kb Hind III fragment region are readily observed in many samples.

Besides homozygous internal deletion in this region, other types of changes including homozygous internal deletion, homozygous 3' deletion, amplification, or combination of these were also observed. These structural aberrations in human breast tumors bear striking similarity to those found in retinoblastoma. Using Southern blot analysis, nine of the nineteen human breast tumors, one human melanoma, ATCC cell line Sk-Mel-5, two osteosarcoma and one fibrosarcoma showed structural aberrations. The percentage of breast tumor cell lines with structural changes is therefore similar to that of retinoblastoma.

Example 3

Diagnosis is done on the tumor and/or the constitutional cells. Whenever tumor material is available, they are tested first for structural defects in the Rb gene by any or all of the techniques described herein. Once the mutation is identified, diagnostic effort is focused in the mutated region and applied on the constitutional cells. For an example. both copies of the Hind III 7.5kb DNA fragments are missing from the tumor DNA of LA-RB74. This structural defect is inherited in the constitutional cell of the patient because one copy of the Hind III 7.5kb is missing in these cells. Therefore, for diagnosis of predisposition to the development of retinoblastoma and related diseases in the immediate relative of the proband, one will focus on detecting the presence or absence of the Hind III 7.5 kb DNA fragment.

In the absence of tumor material, the detection of Rb structural aberration is done on the constitutional cells. In addition, where there is a familial history of retinoblastoma and/or osteosarcoma, the inheritance of polymorphic Rb alleles is searched for and compared to the incidence of Rb tumor in the family.

The fact that structural changes within the Rb locus could be readily detected with the novel cDNA clone in approximately 40% of the retinoblastomas as well as in fibroblasts from some bilateral patients, makes this probe quite useful in prenatal diagnosis. One skilled in the art will readily recognize that as the complete genomic locus is analyzed and more probes and methodologies become available, considerably more abnormalities should be detected.

This cDNA probe should be immediately useful in prenatal diagnosis in families having a parent with a detectable structural abnormality of the Rb gene such as those individuals from whom tumors LA-RB74, LA-RB151, and LA-RB128B or the fibroblast line RB120F were derived. If similar changes were found in fetal cells, this would indicate that the fetus inherited the Rb susceptibility gene. Such a fetus almost certainly would develop retinoblastoma and be at much higher risk to have other malignancies, particularly osteosarcoma, in later years if the pregnancy reached term.

The cDNA probe could also allow one to identify some of the estimated 15% of individuals who have unilateral sporadic Rb but who unfortunately have the hereditary form of the disease.

It is also clear that the Rb gene plays a role in the development of the above-mentioned tumors. The use of the cDNA clone of the Rb gene for the detection of gene defects can therefore be done with other malignancies, including human breast tumors, human melanomas, osteosarcomas, fibrosarcoma, soft tissue sarcoma and small-cell lung carcinoma.

The methods, procedure and techniques described herein are presently representative of the preferred embodiments, are intended to be exemplary, and are not intended as limitations on the scope.

**Claims**

1. A method of detecting tumors containing a retinoblastoma gene defect, comprising the steps of:
    hybridizing a retinoblastoma gene probe to DNA extracted from a target tissue; and
    determining binding of the probe to said DNA, wherein said tumors are selected from the group consisting of fibrosarcoma, soft tissue sarcoma, melanoma, small-cell carcinoma of the lung and breast carcinoma.

2. The method of claim 1, wherein the target tissue is non-tumor tissue.

3. The method of claim 1 or claim 2, wherein the retinoblastoma gene probe is labelled.

4. The method of any one of claims 1 to 3, wherein the retinoblastoma gene probe comprises a cDNA probe of up to about 4.6 kb which is hybridizable to the retinoblastoma gene.

5. The method of claim 4, wherein the retinoblastoma gene probe comprises a cDNA probe of about 0.9 kb which is hybridizable to the retinoblastoma gene and which is an Eco R1 fragment of the cDNA clone of about 4.6 kb.

6. The method of claim 4, wherein the retinoblastoma gene probe comprises a cDNA probe of about 3.8 kb which is hybridizable to the retinoblastoma gene and which is an Eco R1 fragment of the cDNA clone of about 4.6 kb.

**Patentansprüche**

1. Verfahren zum Nachweis von Tumoren, die einen Retinoblastomgen-Defekt aufweisen, umfassend die folgenden Schritte:

   Hybridisierung einer Retinoblastomgen-Sonde mit DNA, die aus dem Zielgewebe extrahiert wurde; und

   Bestimmung der Bindung der Sonde an die DNA, wobei die Tumoren ausgewählt sind aus Fibrosarcom, Weichteile-Sarcom, Melanom, kleinzelligem Lungencarcinom und Brustcarcinom.

2. Verfahren nach Anspruch 1, wobei das Zielgewebe ein Nicht-Tumorgewebe ist.

3. Verfahren nach Anspruch 1 oder 2, wobei die Retinoblastomgen-Sonde markiert ist.

4. Verfahren nach einem der Ansprüche 1 bis 3, wobei die Retinoblastomgen-Sonde eine cDNA-Sonde mit bis zu etwa 4,6 kb umfaßt, welche mit dem Retinoblastomgen hybridisierbar ist.

5. Verfahren nach Anspruch 4, wobei die Retinoblastomgen-Sonde eine cDNA-Sonde von etwa 0,9 kb umfaßt, welche mit dem Retinoblastomgen hybridisierbar ist und ein Eco R1-Fragment des cDNA-Clons von etwa 4,6 kb ist.

6. Verfahren nach Anspruch 4, wobei die Retinoblastomgen-Sonde eine cDNA-Sonde von etwa 3,8 kb umfaßt, welche mit dem Retinoblastomgen hybridisierbar ist und ein Eco R1-Fragment des cDNA-Clons von etwa 4,6 kb ist.

**Revendications**

1. Procédé de détection des tumeurs contenant un défaut du gène du rétinoblastome, comprenant les étapes consistant à :
   hybrider une sonde du gène du rétinoblastome à l'ADN extrait d'un tissu cible, et
   déterminer la liaison de la sonde audit ADN, dans lequel lesdites tumeurs sont choisies dans le groupe formé par les fibrosarcomes, les sarcomes à tissus mous, les mélanomes, les cancers du poumon à petites cellules et les cancers du sein.

2. Procédé selon la revendication 1, dans lequel le tissu cible est un tissu non tumoral.

3. Procédé selon la revendication 1 ou la revendication 2, dans lequel la sonde du gène du rétinoblastome est marquée.

4. Procédé selon l'une quelconque des revendications 1 à 3, dans lequel la sonde du gène du rétinoblastome comprend une sonde d'ADNc atteignant environ 4,6 kb qui peut être hybridée au gène du rétinoblastome.

5. Procédé selon la revendication 4, dans lequel la sonde du gène du rétinoblastome comprend une sonde d'ADNc d'environ 0,9 kb qui peut être hybridée au gène du rétinoblastome et qui est un fragment Eco R1 du clone d'ADNc d'environ 4,6 kb.

6. Procédé selon la revendication 4, dans lequel la sonde du gène du rétinoblastome comprend une sonde d'ADNc d'environ 3,8 kb qui peut être hybridée au gène du rétinoblastome et qui est un fragment Eco R1 du clone d'ADNc d'environ 4,6 kb.

EP 0 364 490 B1

FIG. IA

FIG. IB

FIG. 2

EP 0 364 490 B1

FIG. 3A

FIG. 3B

EP 0 364 490 B1

**1 2 3 4 5 6 7 8 9 10 11 12 13 14 15 16 17 18 19 20**

Kb

-4.7

-1.3

*FIG. 4*

FIG. 5

HindIII/PGH2

FIG. 6

FIG. 7

FIG. 8

FIG. 9

EP 0 364 490 B1

a

5'TGGGCGGAT TAAT TCCCGATCCAGGCATCCTAGATG - - - - -3'

3ACCCGCCTAAT TAAGGGCTAGGTCCGTAGGATCTAC - - - - -5'

5--CT TAATCATCCTCTCAACTCAT TACCGGT - - -3

3--GAATTAGTAGGACAGT TGAGTAATGGCCA--5

b

5' ————————————————————————————————— 3'

3' ————————————————————————————————— 5'

INTRON      SPLICE
JUNCTION      EXON      SPLICE
JUNCTION    b    INTRON

*FIG. 10*

FIG. 11